# EUROPEAN PATENT APPLICATION

(11) **EP 1 609 415 A1**
(43) Date of publication of application: **28.12.2005**
(21) Application number: 04102904.2
(22) Date of filing: 23.06.2004
(51) Int. Cl.: A61B 5/0432

(54) **Method and a device for recording signals**

(71) Applicant: INSTRUMENTARIUM CORPORATION, 00510 Helsinki (FI)
(72) Inventor: Meriläinen, Pekka, 00660, Helsinki (FI)
(74) Representative: Valkeiskangas, Tapio Lassi Paavali

(57) **Abstract**

The invention relates to a method and a device for recording at least one signal from an object. The method comprises the following steps. Providing a recording device (1) comprising at least one electrode and/or a sensor, a preamplifier, microprocessor, A/D-converter, memory and a power source, attaching the recording device on the surface of the object (2), activating data collection from the signal obtained from the object (2) when the recording device (1) is in contact to the surface, letting the data collection run continuously for a preset time characteristic for the purpose of the study and returning the recording device (1) to a service provider (3,4) for calculating and analyzing the results off-line with computer programs.

## Description

The invention relates to a method for recording at least one signal from an object. The invention relates further to a recording device for recording at least one signal from an object.

Recording of signals is one of the basic measurements in different fields, for example in diagnostics and follow-up of several common or chronic diseases of human beings and animals. Diagnostics and follow-up of several common and/or chronic diseases is currently mostly based on measurements of certain physiological parameters of human body or animal body under laboratory conditions. Many of the measurement set-ups are complicated and may interfere or stress the subject and alter the results in a way leading to false diagnosis and wrong prescription of medication. A typical example is non-invasive blood pressure measurement where results obtained at doctor's office have been found to be typically up to 20% higher compared to pressures measured by the patient with modern devices in home conditions. Another important aspect is that drawing conclusions from short spot check type of laboratory measurements is in most cases more uncertain than a longer continuous measurement preferably up to 12 or 24 h or even more, which can reveal the level and variations of the physiological parameters of interest under the course of everyday normal day and night. Additionally, it is often advantageous to measure several relevant parameters simultaneously to help understanding the results, like for example looking at correlation between heart rate and physical activity.

Some light portable or wearable monitoring methods already exist or have been proposed for diagnosis and/or monitoring chronic diseases at home or under field conditions (e.g. US Patent Specification No. 6,254,551) as well as in sport and fitness related applications. Lot of efforts have been devoted for developing wireless communication methods from a small measurement unit to a monitor or PC for recording and analyzing the data. However, the mere presence and touch of many current solutions may trouble the patient and prevent completing of long term measurements or distort the results for example in studies of sleep disorders.

Portable data loggers of heart signals called "Holter" recorders of ECG have been in use for long time. Collecting ECG by an electrode set integrated in a band around the chest and transmitting the pulse signal wirelessly to a processing device of a wrist watch type is also in routine use in consumer pulse rate meters. US 6,254,551 disclose an apparatus for monitoring a mechanically transmitted physiological signal from the body and for processing the results. It is based on collecting composite signal from two or more sensors to be decomposed to "partial spectra" in later processing by a standard computer. It is designed for being fitted onto the subject's chest without skin contact and provided with a cable or wireless link to transmit data from the sensor body to the data collection device.

The object of the present invention is to obtain a method and a device by means of which the disadvantages of the prior art can be eliminated. This is achieved with the invention. The method of the invention is characterized by the steps: providing a recording device comprising at least one electrode and/or a sensor, a preamplifier, microprocessor, A/D-converter, memory and a power source, attaching the recording device on the surface of the object, activating data collection from the signal obtained from the object when the recording device is in contact to the surface, letting the data collection run continuously for a time characteristic for the purpose of the study and returning the recording device to a service provider for calculating and analyzing the results off-line with computer programs. The recording device of the invention is in turn characterized in that the recording device comprises a base member reliably and detachably attachable on the surface of the object, at least one electrode or/and a sensor attached on the base member, a preamplifier, microprocessor, A/D-converter, memory, a power source attached on the base member and wirings to pick up, to convert and to transfer data from the signal obtained from the object to the memory, activating means for activating the data collection from the signal when the base member is in contact with the surface of the object and an interface element attached on the base element for transferring the data out of the memory.

The advantage of the invention is that the invention can be used in many different measurements. The device of the invention is also very simple and extremely easy-to-use for a patient. Simple construction enables that the invention can be materialized without excessive costs.

In the following the invention will be described in greater detail with reference to the accompanying drawings, in which
Figure 1 shows principally the basic steps of the method of the invention,
Figure 2 shows principally an optional location of the recording device,
Figure 3 shows one embodiment of a recording device of the invention,
Figure 4 is another view of the embodiment shown in Figure 3 and
Figure 5 shows a detail of the embodiment shown in Figures 3 and 4.

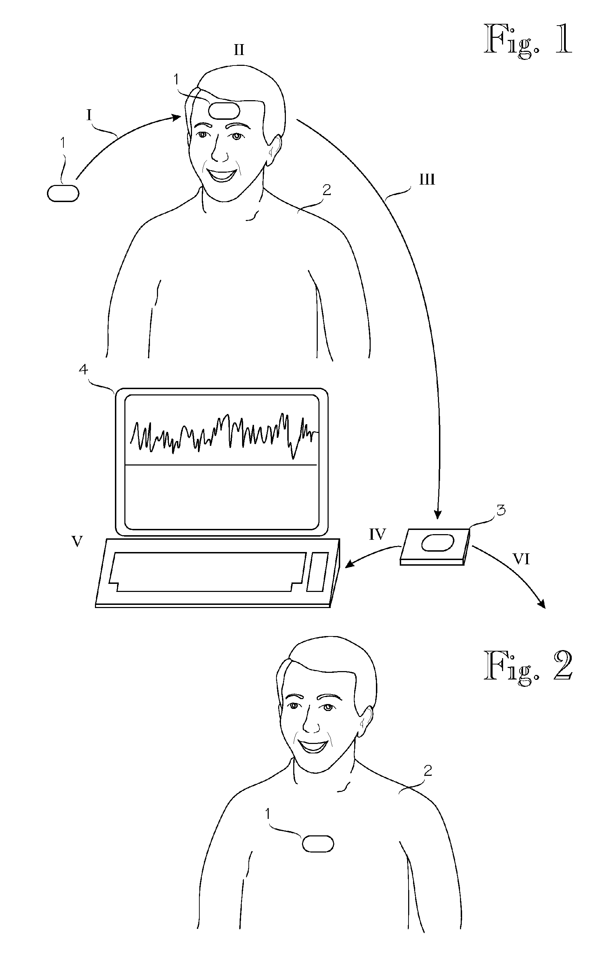

Figure 1 shows the basic steps of the present invention. Reference number 1 shows a recording device of the invention. Reference number 2 shows a patient. The recording device 1 is placed on the forehead of the patient 2. The recording device is attached on the forehead by using an adhesive paled on the underside of the base member. This step is shown by an arrow I in Figure 1. Data is collected from a signal obtained from the patient 2. This step is shown by number II. Data is collected for a preset time. The recording device 1 is detached and taken to a memory reader 3. This step is shown by an arrow III. The data is downloaded to a computer 4, preferably a PC. This step is shown by a number IV. The memory reader 3 and the computer 4 can be seen here as parts of a service provider for calculating and analyzing the results. In the embodiment shown said service provider is a medical service provider. Advanced calculations and analyzes of the data are carried out in the PC This step is shown by a number V. The recording device can be thrown away as shown by an arrow VI.

When data is collected from a patient it is possible to collect data from one signal or data from several signals simultaneously. Data collection can also be repeated at certain intervals, and the data collected can be compared to the data collected later. In other words data can be for example collected on day time and on night time and the results recorded on day time can be compared to the results recorded on night time. It is also possible to collect data when the patient is awake and when the patient is asleep and to compare the results obtained. It is further possible to collect data when the patient is under physical exercise and when the patient is not under physical exercise and compare the results obtained under physical exercise and under rest.

One of the basic thoughts in the invention is a concept of an attachable and most preferably disposable recording device 1 miniaturized down to level where it does not interfere the patient 2 more than a piece of standard plaster taped on the skin. The modern transducer technology utilizing microelectronics and micro electro mechanical systems (MEMS) makes it possible to design miniature sensors attachable directly on the skin of the subject for picking up physiological signals of interest. The recording device 1 can be characterized as an integrated digital data logger attached on the skin of the patient 2 in appropriate location related to the organ being under investigation. In Figure 1 the recording device is attached on the forehead of the patient 2. Figure 2 shows an alternative recording device site, i.e. the device 1 is attached on the chest of the patient 2.

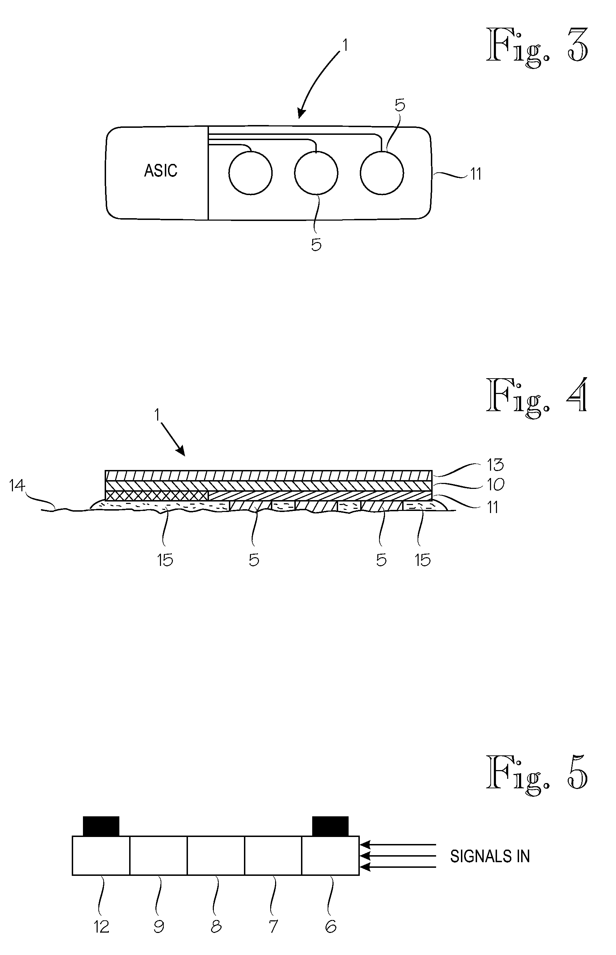

The recording device 1 comprises at least one electrode or/and sensor 5, preamplifier 6, microprocessor 8, A/D-converter 7, memory 9 and power source 10, for example a battery. Said components are placed on a base member 11, which is preferably a flexible substrate layer, for example a tape member. Power source 10 can preferably be a flexible battery. These details are shown in Figures 3, 4 and 5. Preamplifier 6, microprocessor 7, A/D-converter 8 and memory 9 are combined into an ASIC chip (Application Specific Integrated Circuit) in the embodiment of the Figures. Said ASIC chip comprises also an interface element 12 for transferring the data out of memory 9 as shown in Figure 5. Electrodes or sensors 6 can be for example ECG-electrodes, EEG-electrodes, microphones, pressure and acceleration sensors etc. as described later.

The recording device 1 can be activated to start measuring when a protective layer 13 is removed right before attaching the sensor on the skin of the patient. The skin of the patient is shown by number 14 in Figure 4. The protective layer 13 can be placed to cover the upper side of the recording device 1 as shown in Figure 4. The protective layer 13 can however be placed to cover the adhesive layer 15 of the device 1 whereby the protective layer 13 is removed right before attaching the device on the skin.

The recording device 1 can be programmed for stopping measurement and getting deactivated after a fixed period like 8, 12, 16 or 24 h. The sensors of highest interest in this context are ECG- and EEG-electrodes, microphones, pressure and acceleration sensors. The concept would for example make it possible to develop a disposable sleep laboratory including recording of sleep phases and quality of sleep by EEG analysis and/or sleep apnea and snoring by a microphone. It can also be employed in heart rate analysis and pulmonary medicine for following wheezing and other lung sounds of asthma patients. EEG electrode or EEG-electrodes can be used for example for diagnosing the electrical activity of the brain and/or the electrical activity of muscles covering the head surface. ECG-electrode or ECG-electrodes and/or a microphone or microphones and/or an acceleration sensor or acceleration sensors can be used for example in studying the performance of heart. A microphone or microphones and/or an acceleration sensor or acceleration sensors attached on the chest can be used in studying the lung function based on analysis of lung sounds.

The concept of the invention is not intended for continuous real time monitoring of for example physiological parameters, but for profound off-line analysis of the data with most sophisticated signal processing algorithms.

The method and apparatus of the invention differs from the prior art in several fundamental aspects. It is intended for use in direct contact to the surface of the object measured, for example in direct skin contact typically on the forehead or on the chest of the patient. It includes a means for processing the raw electrical signal into digital format at high sampling frequencies and transferring the data into a solid state memory immediately on the same physical component entity as the sensor itself. Also a miniature battery with a life time optimized for the intended length of measumerent is integrated on the same substrate. The entire unit is designed to be fabricated in mass production and intended to be used as a disposable item. The collected data contents on the memory chip can be down loaded into the external device performing the final signal analysis by standard means like for example a USB port.

A typical example of applications of the invention would be miniaturizing a method used for measuring depth of anesthesia employing a mathematical method called entropy on the EEG signal collected from one channel from the forehead (US Patent Application No. 20030167019) and optimizing these algorithms for analyzing various features of natural sleep as well. For the patient's point of view the device would be a piece of plaster to be attached on the forehead before going to the bed, removing it in the morning and being returned to the doctor who has prescribed this measurement in order to diagnose the type of a possible sleep disorder. Then if certain drugs will be prescribed to treat the disorder, new measurements can be performed in same manner to see the effectiveness of the drug care. Same concept can be used in diagnosis of asthma by using a microphone or several microphones instead of electrode/sensor or electrodes/sensors in the plaster body. This kind of a tool would be especially optimal in diagnosis and follow-up of children's asthma where the acoustical signals originating from the lungs are more easily accessible than in adults.

An additional type of a sensor, which could be utilized in this concept, is a sensitive one or multi axis acceleration sensor which could be used e.g. in analyzing body movements, heart beats or snoring. It is naturally possible to use several acceleration sensors, too.

The embodiment of the invention described above and in the drawings is by no means intended to limit the invention, but the invention can be modified freely within the scope of the claims. The invention is for example described above by means of an embodiment relating to recording a physiological signal from a human skin. Said embodiment is not however the only possible embodiment, but the invention can naturally be used for example in recording signals from an animal. The invention can also be used in recording signals from not living object, for example in applications where maintaining quality of special food products requires them to be kept continuously under certain ambient conditions, like within a fixed range of temperature and/or humidity, over the entire transport chain.

## Claims

1. A method for recording at least one signal from an object, **char acterized by** the steps:
- providing a recording device (1) comprising at least one electrode and/or a sensor (5), a preamplifier (6), microprocessor (8), A/D-converter (7), memory (9) and a power source (10),
- attaching the recording device on the surface (14) of the object (2),
- activating data collection from the signal obtained from the object (2) when the recording device (1) is in contact to the surface (14),
- letting the data collection run continuously for a time characteristic for the purpose of the study and
- returning the recording device (1) to a service provider (3,4) for calculating and analyzing the results off-line with computer programs.

2. The method as claimed in claim 1, **characterized in that** data from several signals are collected simultaneously.

3. The method as claimed in claim 1 or 2, **characterized in that** the data collection are repeated at certain intervals.

4. The method as claimed in claim 3, **chracterized in that** the data collected is compared to the data collected later.

5. The method as claimed in any of the claim 1 - 4,**characteri zed in that** the signal recorded is a physiological signal.

6. The method as claimed in claim 5, **characterized in that** the surface (14) on which the recording device is attached, is the outer surface of a living organism.

7. The method as claimed in claim 6, **characterized in that** the outer surface of a living organism is a human skin.

8. The method as claimed in claim 1, **characterized in that** the service provider (3,4) is a medical service provider.

9. The method as claimed in claim 1, **characterized in that** the data collection is let to run continuously for a preset time.

10. A recording device for recording at least one signal from an object, **characterized in that** the recording device comprises
- a base member (11) reliably and detachably attachable on the su r-face (14) of the object (2),
- at least one electrode or/and a sensor (5) attached on the base member (11),
- a preamplifier (6), microprocessor (8), A/D-converter (7), memory (9), a power source (10) attached on the base member and wirings to pick up, to convert and to transfer data from the signal obtained from the object (2) to the memory (9),
- activating means (13) for activating the data collection from the signal when the base member (11) is in contact with the surface (14) of the object (2) and
- an interface element (12) attached on the base element for transferring the data out of the memory (9).

11. The recording device as claimed in claim 10, **characteriz ed in that** the base member (11) is a flexible substrate member.

12. The recording device as claimed in claim 10 or 11,**characte rized in that** the activating means (13) is a protective layer covering the base member (11).

13. The recording device as claimed in any of the claims 10, 11 or 12, **characterized in that** the electrodes or/and sensors (5) comprise ECG electrodes or/and microphones, or/and acceleration sensors.

14. The recording device as claimed in any of the claims 10 - 13, **characterized in that** the recording device (1) is a disposable device.

15. The recording device as claimed in claim 10, **characteriz ed in that** the base member (11) is reliably and detachably attachable on the outer surface (14) of a living organism.

16. The recording device as claimed in claim 15, **characteriz ed in that** the outer surface of a living organism is a human skin.
